(19) Europäisches Patentamt

European Patent Office

Office européen des brevets

(11) Publication number: **0 252 594 B1**

(12) **EUROPEAN PATENT SPECIFICATION**

(45) Date of publication of patent specification: **09.03.94**    (51) Int. Cl.⁵: **A61N 1/00**

(21) Application number: **87304576.9**

(22) Date of filing: **22.05.87**

Divisional application 93100472.5 filed on 22/05/87.

(54) **Portable electro-therapy system.**

(30) Priority: **23.05.86 US 866877**

(43) Date of publication of application:
**13.01.88 Bulletin  88/02**

(45) Publication of the grant of the patent:
**09.03.94 Bulletin  94/10**

(84) Designated Contracting States:
**AT BE CH DE ES FR GB GR IT LI LU NL SE**

(56) References cited:
**EP-A- 0 056 697        EP-A- 0 103 491**
**WO-A-83/02901        WO-A-85/00293**
**WO-A-85/02547        WO-A-86/01733**
**GB-A- 1 390 732        US-A- 3 952 751**
**US-A- 4 467 809        US-A- 4 535 775**
**US-A- 4 548 208        US-A- 4 556 051**

(73) Proprietor: **THE TRUSTEES OF THE UNIVERSITY OF PENNSYLVANIA**
**Mellon Bank Building**
**133 South 36th Street**
**Suite 419**
**Philadelphia Pennsylvania 19104(US)**

(72) Inventor: **Griffith, Neil**
**3959 Sequoia Street**
**San Diego California 92109(US)**
Inventor: **BRIGHTON, Carl, T.**
**14, Flintshire Road,**
**Malvern, Pennsylvania 19355(US)**
Inventor: **POLLACK, Solomon, R.**
**3213, Ayr Lane,**
**Dresher, Pennsylvania 19025(US)**
Inventor: **PIENKOWSKI, David**
**211, Ravenwood Road,**
**Exton, Pennsylvania 19341(US)**

(74) Representative: **Wilson, Nicholas Martin et al**
**Withers & Rogers**
**9 The Parade**
**GB-Leamington Spa, Warwickshire CV32 4DG (GB)**

**EP 0 252 594 B1**

## Description

A variety of biochemical events, particularly changes in ion transport, protein synthesis and the like are involved in the repair of fractured bones. It has long been recognized that properly applied electro-therapy signals can stimulate bone growth in the vicinity of fresh fractures and non-union fractures, and apparently do so by initiating or stimulating the requisite biochemical changes. Extensive research has been conducted in both experimental animal studies and human clinical trials utilizing various specific waveform formats for such treatment, including invasively-coupled, direct-current devices; capacitively-coupled, symmetric and asymmetric waveforms, and electro-magnetically coupled asymmetric waveforms. Excellent technical reviews of this field are J. A. Spadaro's Bioelectric Stimulations of Bone Formation: Methods, Models and Mechanisms, in the "Journal of Bioelectricity", Volume 1 (1), p. 99, 1982; and the Orthopedic Clinics of North America Symposium on Electrically Induced Osteogenesis, W.B. Saunders Corp. 1984.

All currently used electro-therapy therapy techniques have one or more limitations. For example, invasive techniques have the potential of increasing the risk of infection, and unpredictable and potentially long-term side effects. Capacitively-coupled systems are limited by the fact that when operated at safe voltage levels, they require electrical connection between capacitive plates and the skin in the area surrounding the fracture site. Conductive jellies are typically employed and, by their nature, do not lend themselves to long-term installation. Electromagnetically, inductively-coupled techniques have required high power consumption waveform generation devices and bulky coil configurations that compromise the patient's ability to function normally outside of the clinical environment. Representative U.S. patents describing the above devices are 4,535,775, inventors Brighton and Pollack; 4,667,809, inventor Brighton; 4,467,808, inventors Brighton and Pollack; 4,266,532, inventor Ryaby; 3,952,751, inventor Yanger; 3,893,462, inventor Manning; and 3,890,953, inventors Kraus and Viehbach.

It generally takes bone fractures, particularly non-union fractures, many weeks or months to heal, and this is true even with the aid of electro-therapy where it has been tried as an adjunct treatment in an experimental setting. Because the presently utilized electro-therapy devices are, with a few exceptions, not truly portable, if the patient is to benefit from electro-therapy, he must have ready access to a source of electric power to effect treatment. Considering the time required for a bone to heal, this constraint is particularly annoying on a day to day basis, and requires that a patient constantly interrupt his daily routine for treatment, which may in turn cause failure of the patient to comply with the required protocol. Therefore, it is apparent that it is desirable to produce a device having the effective features of the devices currently in use but lacking their undesirable features, particularly their power wasting aspects. By creating a more power-efficient electro-therapy device it is possible to considerably reduce the size of the electro-therapy machines, hence permitting the construction of a completely portable device that allows the user to go about his daily routine without being tethered to a source of electric power.

A few inventors have appreciated the practical advantages of having a portable electro-therapy device. It is important to note that portability in the art is taken to mean a device readily carried by the patient without cumbersome support aids, and particularly connotes devices less than two pounds in weight, and no larger than a small Instamatic® type camera. U.S. Patent 4,432,361 describes a portable device that has self monitoring features thereby allowing the patient to ascertain its operational status without having to have it checked by a physician, or another person skilled in the use of the device. This invention is an improvement over that described in U.S. patent 3,842,841 which does not have the desirable self-monitoring features. Another portable electrotherapy device is described in U.S. Patent 4,574,809. It shows a device suitable for integration into an orthopedic cast with a signal generator removably mounted in the cast.

In U.S. Patent 4535775 there is disclosed a method for treatment of bone fractures by applying electrodes to the skin of a patient in the vicinity of the break. The signal applied to the electrodes may be an alternating voltage having a waveform that is symmetrical about the axis, a frequency in the range of 20 to 100 kHz and a value ranging from about 2 to 10 Volts peak-to-peak. The electrodes may be energized either continuously or intermittently.

International Publication WO-A-85/02547 discloses a technique for non-invasive treatment whereby a magnetic field is non-invasively applied to a treatment area by energizing a coil with electromagnetic pulses. At least two and possibly a plurality of such pulses are applied during the active pulse interval.

However, both those prior devices require a relatively large power source and therefore are not practical as portable arrangements. On the other hand the present invention is able to use a low power source since, as claimed, the voltage cycles comprise a train of bursted pulses having a pulse width of 0.5-20 microseconds.

This patent describes a portable electro-therapy device having considerable advantages over prior devices. In an exemplary embodiment of the invention, the deficiencies of prior art systems are overcome in a system in which an effective treatment signal is produced in a low power consumption device, and then efficiently coupled to the treatment site by transducers that may be conformed to the external body line contours in the vicinity of the treatment site so that the entire system lends itself to portable, ambulatory use.

According to the present invention there is provided a portable apparatus for electro-therapy of bone fractures and for stimulating tissue healing at a treatment site, said apparatus having a source of electric power connected to means for converting the electric power into a series of amplitude-symmetric voltage cycles and transducer means for receiving said symmetric voltage cycles characterized in that:

the voltage cycles comprise a train of bursted pulses having a pulse width of 0.5-20 microseconds;

the burst frequency is in the range of 5-25Hz; the burst width is 1-10 milliseconds; and

the transducer means is positionable so that an electromagnetic field at the treatment site is generated by applying the electric power to the transducer.

Prior to the Applicant's invention, it was generally assumed that the body did not respond to short duration symmetric electromagnetic pulses, resulting in the development of bulky circuit devices for generating the long-duration pulses thought necessary for effective treatment. Applicant has discovered an hithertofore unrecognized feature of the biology of healing realized by effectiveness of the device encompasses healing of damaged tissues generally. Symmetrically shaped pulses of a relatively short pulse width, and when grouped into a burst of pulses followed by a quiet time, are as efficacious as devices applying other electrical parameter. This realization has permitted the design of a device using several orders of magnitude less power than required by existing devices. This finding, coupled with conformal transducer designs for delivering the low power signal the damaged tissue site, permits the development of a truly portable electro-thereapy device.

A variety of transducers of specific designs can contribute to the overall energy efficiency of the subject device. However in an exemplary embodiment of the invention, the conformal assembly complex comprises a solenoidal coil of varying turns applied around a limb and connected to a power source to generate signals at the treatment site.

As applied to healing of bone fractures, the application of the invention described herein in no way affects or interferes with present treatment protocols. Thus, the physician is given a treatment option especially for those fractures, for example, non-union fractures, which, by experience and analysis, have been determined to be most likely to require surgery or other invasive procedures.

**BRIEF DESCRIPTION OF THE DRAWINGS**

Figure 1 shows an effective electro-therapeutic signal.

Figure 2 relates the amplitude of an effective signal with the stiffness ratio when $\Delta t_{PW}$ = 5 $\mu$sec, $\Delta t_{BW}$ = 5 msec and $f_{BW}$ = 15 Hz.

Figure 3 compares the energy efficiencies of various coil transducers.

Figure 4 displays a solenoid transducer positioned about a patient's leg with a fractured tibia.

Figure 5 reveals a block diagram of the subject invention.

Figure 6 depicts a perspective view of a conformal magnetic dipole transducer.

Figure 7 is an enlarged sectional view taken on line 2-2 of Figure 6; and

Figure 8 is an enlarged sectional taken on line 3-3 of Figure 7.

Figure 9 depicts the lines of force of the magnetic field established by a shielded dipole.

Table I shows that an effective signal is realizable down in the range of 0.5 $\mu$sec pulse width.

Table II shows stiffness ratios obtained at varying pulse widths over the range 2-10 $\mu$sec.

Table III shows displays stiffness ratios obtained at varying amplitudes using 5 $\mu$sec pulse width.

Table IV shows the calculated ranges of useable hours of the electro-therapeutic device as a function of different batteries, coil number, and signal pulse width.

**DETAILED DESCRIPTION OF THE INVENTION**

It has been discovered that an electric signal of particularly unique parameters is able to stimulate healing of bone fractures, and damaged tissues, especially bone fractures that do not readily heal in the absence of treatment such as non-union fractures. However, the subject invention is equally effective in healing delayed unions and failed fusions.

The portable electro-therapy device described herein is based on experimental studies showing the therapeutic effectiveness of an electro-magnetic signal having a symmetric waveform consisting of a train, having positive and negative phases, of pulses and of a particular range of burst widths, pulse widths, peak

3

amplitudes and frequencies. A key feature of this signal is that it consumes nearly an order of magnitude less energy than current state-of-the-art devices using different signals. In part, this is because we have shown that the pulse width of an efficacious signal can be reduced down in the range of 0.5 microseconds and still effect tissue healing. The electro-magnetic field, and associated power density established within the tissue is proportional to dB/dt, or the rate of change of the magnetic flux density, and thus is independent of pulse width for dB/dt = constant. Since the power required to generate a signal is a function of the pulse width squared, the power needed to establish an efficacious signal can be reduced by narrowing the pulse width. Hithertofore, this aspect of the electrobiology of tissue healing, particularly bone healing, has not been appreciated.

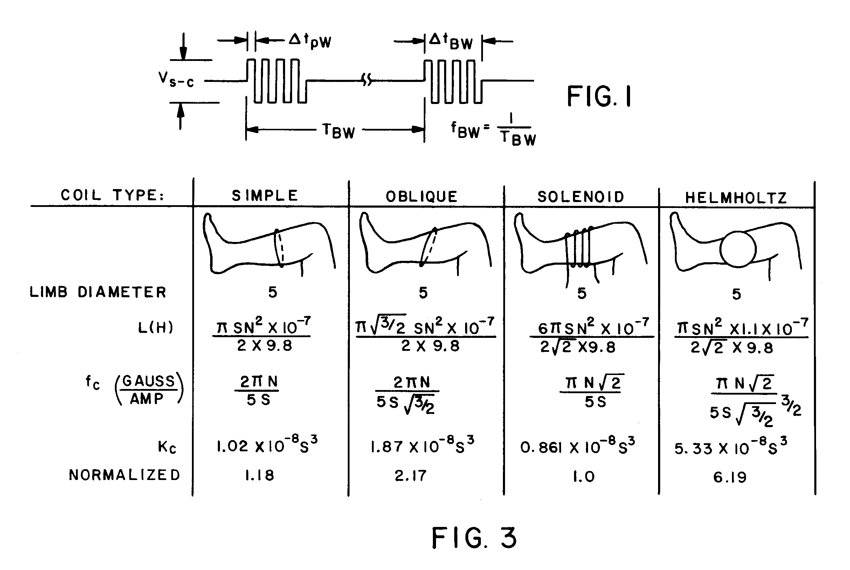

Biological studies were conducted on an animal model system wherein the efficaciousness of the pulsed signal was established. The most reliable animal model for these studies has proven to be the rabbit fibular system as described by C.T. Brighton et al. in the Journal of Orthopedic Research, Volume 3, No. 3, 1985. In this system rabbits undergo a mid-shaft transverse osteotomy of one fibula, after which a suitable transducer connected to a power supply was installed over the fracture. Both experimental and control animals were treated with the signal shown in Figure 1 where $V_{s-c}$ is the search coil voltage; $\Delta t_{PW}$ is pulse width; $\Delta t_{BW}$ is burst width and $f_{BW}$ is burst frequency. We have found that a $\Delta t_{PW}$ between 2 and 10 microseconds is therapeutically effective, with 5 microseconds being particularly effective. Presently used devices generate $\Delta t_{PW}$ in the ranges of 20 to 300 microseconds for asymmetric signals.

While we experimentally have shown that a $\Delta t_{PW}$ of 2-10 $\mu$sec is effective, it will be readily appreciated by those skilled in the art that a $\Delta t_{PW}$ of 0.5-20 microseconds is therapeutically acceptable. This is anticipated from simple theoretical considerations. For instance, activation of the 30 cellular machinery involved in bone or tissue repair by electro-magnetic radiation requires delivery of a signal to the injured site having defined time constants for burst, width and burst frequency. In order to realize this, it is necessary for the signal to traverse healthy tissue to reach the injured site, and thus not be attenuated before doing so. This in turn suggests that the time constants associated with the magnetic, electric, chemical, and electro-diffusion effects caused by the signal exhibit particular time constants. It will be appreciated, referring to Table I (from "Electric Fields, Forces, and Flows in Biological Tissue," Al Grodzinsky, MIT, July 1985) that the magnetic "diffusion" equation assures that below 100 MHz that the magnetic field completely penetrates through to the injured site. For electric "diffusion", penetration of the bone by the displacement current density remains low until 1 MHz. Further, the viscous flow of interstitial fluids in the canaliculi can follow frequencies up to one MHz. In contrast, however, mechanical stress frequency responses attenuate after 500 Hz. Based on this brief analysis, it is apparent that extrapolition downward from 2 microsecond pulses widths to 0.5 microseconds is anticipated to produce therapeutically effective results.

Electro-therapy was continued for a period of 16 days during which time $V_{s-c}$, $\Delta t_{PW}$ and duty cycle were varied. Following treatment, both control and experimental animals were sacrificed and the fractured fibula excised. The fibula were mechanically tested for 3 point bending stiffness in a CGS Lawrence testing apparatus as described by Brighton, discussed supra, and the maximum resistance to bending measured for all the fibulae. The stiffness ratios of the fractured to intact fibulae of the electrically stimulated rabbits was determined and compared to those of the non-stimulated rabbits.

Briefly, within about 30 minutes after sacrificing the animals, a deformation rate of about 4 mm/min. was used and a parameter, the stiffness ratio determined. The latter is derived by ascertaining the slope of the load-deformation curve, or stiffness, of fractured and intact fibula in the same animal. In this way variation in fibular strength for individual animals is controlled. The stiffness ratios of animals in the experimental group were compared to those of control animals at various pulse widths and amplitudes.

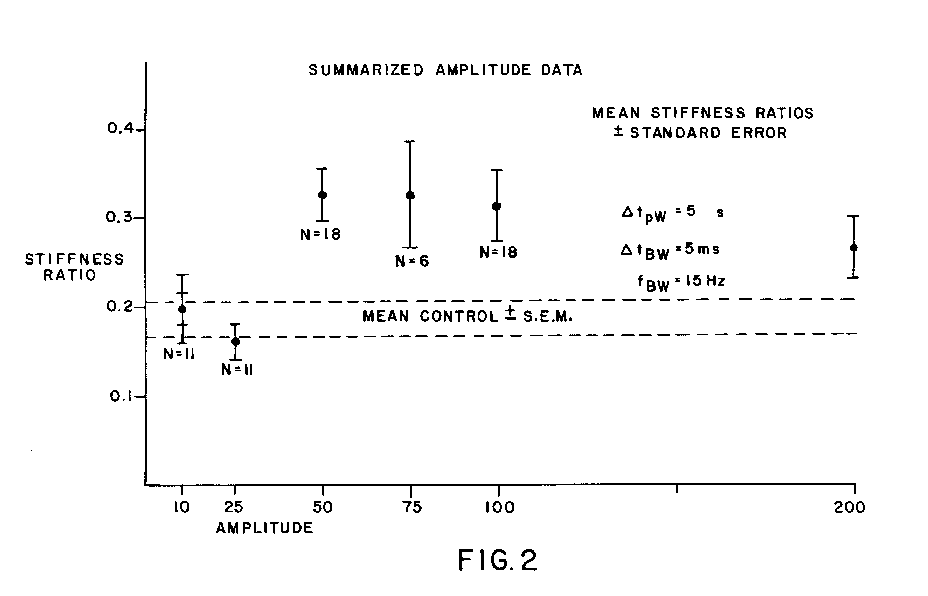

Table II shows that the stiffness ratios obtained are statistically significant, particularly when $V_{s-c}$ is 74 mV, $\Delta t_{PW}$ is 5 $\mu$sec, $f_B$ is 15 Hz, $\Delta t_{BW}$ is 5 msec, and $t_{PW}$ is varied from 2-10 $\mu$sec. Moreover, Table III and Figure 2 reveal that the effectiveness of these parameters is a function of the amplitude of the signal. An amplitude of greater than 25 and less than 200 mV peak is effective with 50-100 being particularly effective, with a search coil having 67 turns and a diameter of 5.8 mm.

While the signal shown above is particularly electro-therapeutically effective, it is to be anticipated that other signals in addition to the simple positive and negative square wave will be efficacious. Thus it should be emphasized that key features of a suitable signal are that it be symmetric, have a narrow pulse width as described above, and, moreover, display a bursted format. Thus it is to be anticipated that rectangular waves, sine waves and other wave forms with these properties will be therapeutically effective.

The above discussion shows that it is possible to effect healing of bone fractures at hithertofore unsuspected low pulse width signals. This finding led us to construct a small portable battery driven device capable of producing an efficacious signal with suitable strength ranging from 3-9 mv/cm at a distance of 2 cm. However, before we could take advantage of our electro-therapy observations which opened the door to portability, it was desirable to combine the signal generator with a more energy efficient transducer means for delivering the signal to the tissue damaged site. A determination of the optimal transducer design necessarily requires a consideration of the power efficiencies of various transducers. Further, partly determinative of the type of transducer that is favored for a particular application in the case of a bone fracture is the nature of the bone fracture sought to be treated. Thus, for deep non-union fractures, particularly those that occur to the femur, a transducer capable of delivering energy through considerable soft tissue is desirable. In contrast, a transducer requiring less power to maintain the same field strength can be employed for bone fractures nearer the skin, e.g. tibia, clavicle.

A coil-type transducer is most preferred for treating deep bone fractures. Consider, for example, that the power needed to be applied to a coil-type transducer (i.e. Helmholtz paired coils, simple coil, simple coil oblique to the fracture or solenoid) is:

$$\textbf{Equation 1}$$

$$P = (1 - n_{REC}) \left( \frac{L}{2} - \frac{1}{f_c} \right)^2 (V_{SC} \cdot 4 \times 10^6)^2 \Delta t_{PW} \Delta t_{BW} f_B$$

where $n_{REC}$ is the fractional energy recovery coefficient, L is the coil inductance, $f_c$ is coil sensitivity, $V_{s-c}$ is search coil voltage, and $\Delta t_{PW}$, $\Delta t_{BW}$ and $f_B$ are pulse width, burst width and frequency respectively. It is apparent, therefore, that a coil constant reflecting the power efficiencies of the various types of coil transducers can be represented as:

$$K_c = \frac{L}{f_c^2}$$

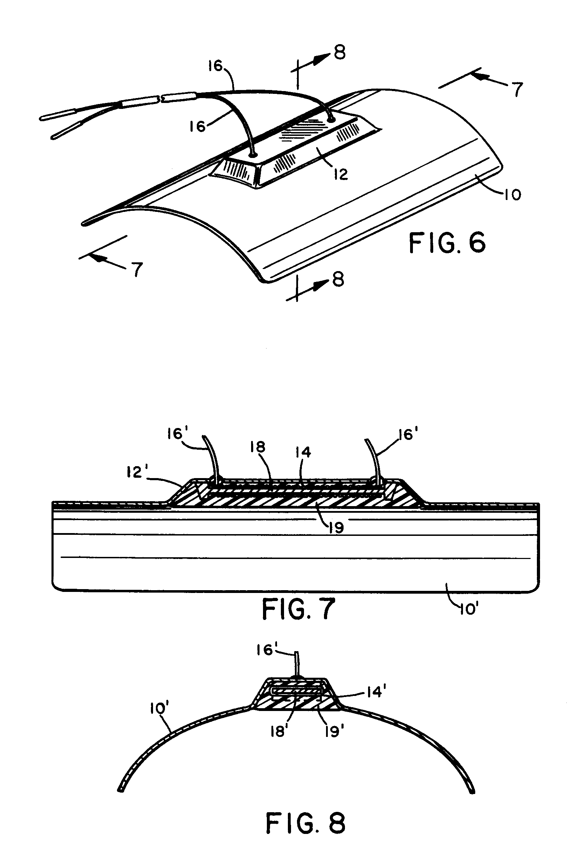

A comparison of $K^c$ for several coil type transducers is shown in Figure 3 reveals that a solenoid coil type transducer is the most energy efficient. Indeed, the order of energy efficient coil transducers is solenoid > simple coil > oblique > Helmholtz.

It will be appreciated that when a conformal solenoid transducer for transmitting the signal shown in Figure 1 is combined with a signal generating device, that the combination is capable of being integrated into a cast or associated therewith. An example is shown in Figure 4. It will be further appreciated that while a conformal solenoid is preferred, that the other coil transducers may also be employed. Thus, a truly portable electro-therapy unit is readily constructed for treating shallow or deep fractures. Figure 5 presents a typical block diagram for this unit.

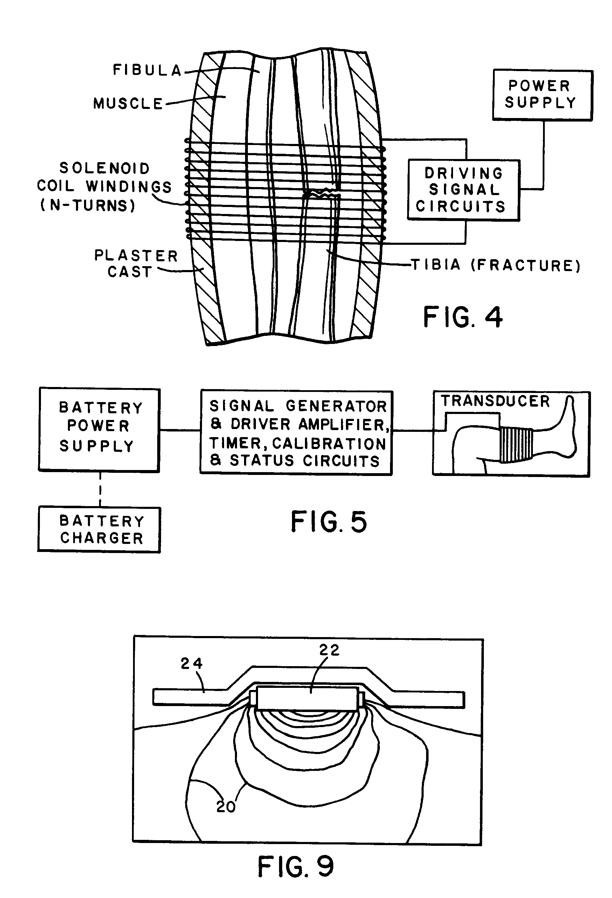

Another exemplary transducer is the conformal, magnetic dipole (CMD) which efficiently establishes shallow, focused fields on relatively superficial fractures, such as the tibia, ulna, radius, clavicle, or scaphoid. Whereas other transducers, simple coils, Helmholtz coils, etc., establish large, power-wasting fields external to the transducer, the CMD "captures" these extraneous fields and redirects them towards the fracture zone, effecting a 20-40% power savings while reducing exposure of the remaining body regions to extraneous fields. Figures 6-8 show an example of a typical conformal magnetic dipole transducer. In Figures 6-8 the transducer exhibits a metal shield 10, 10′ having an uplifted region 12, 12′ for housing the transducer coils 14, 14′. Current is supplied by the wires 16, 16′ and flows through the coils 14, 14′ wrapped about a suitable support element 18, 18′. The latter is held in place by glue, resin or the like 19. Figure 9 shows the lines of force of the magnetic field emitted from the conformal magentic dipole. It will be appreciated that by directing the magnetic field in a preferred direction that there is a considerable savings in energy.

It will be apparent that portability can be realized by integrating, or in some way associating, the transducer with the cast and having the power source and signal generator removed a short distance away. For example, the latter components might be attached to the user's waist belt and connected via leads to the transducer. Alternately, all the components may be attached to, or integrated into the cast. The former situation may be favored when a patient needs constant electrostimulation which may necessitate frequent replacement of batteries. On the other hand, for patients with minor fractures the duty cycle needed for maximum rate of healing may be considerably reduced, and there may be no need to change batteries over the required stimulation period. Here it might be desirable, for convenience to the patient and esthetic purposes, to integrate the entire unit in the cast.

It is anticipated that the power source used to generate the therapeutic signal will weigh less than 2 pounds and be about the size of a medium size hand-held Instamatic® camera.

It will be appreciated that because of the energy efficiency of the electro-therapy device that the user will not have to replace the batteries for at least days, and perhaps not for months. Table IV shows the approximate number of useable hours of a device driven by particular batteries when the device has a solenoid transducer with a diameter of 12.7 cm, an l/d ratio of about 1, and a resistance of 0.63 ohm. Varying these parameters will, of course, effect the size of the batteries needed to achieve treatment. Furthermore, it will be noted that the information in the table was derived using the equation:

**Equation 2**

$$I_B = I_{OH} + \left[ \frac{K_L V_{s-c_{p-p}}^2 \Delta t_{PW}^2}{V_S N^2} (R_g + r/t\,N) - (\Delta t_{BW} \cdot f_{BW}) - \left(\frac{2}{V_S} \times 10^{-3}\right) \right]$$

where:
$I_B$: Batt-Current
$I_{OH}$: Overhead Current
$K_c$: Coil Constant
$\Delta t_{PW}$: = Pulse Width
$N$: Turns/Coil
$V_S$: Battery Voltage
$R$: Driver Circuit, Output Resistance
$r/t$: Resistance/Turn
$\Delta t_{BW}$: Burst Width
$f_{BW}$: Burst Freq
and

$$V_{s-c_{p-p}} = 200 \text{ mv,}$$

$t_{PW}$ = 5 msec, $t_{BW}$ = 5 msec and $f_B$ = 15 Hz.

An additional feature concerning the subject invention will be appreciated by referring to Equation 2 and Table III. It is apparent that "N", or the number of turns comprising the solenoid, is not invariant. By increasing or decreasing the number of turns it is possible to considerably alter the lifetime of the batteries used to drive the device. Thus for a particular application, N will be chosen to best effect the user's needs.

A further embodiment is shown in Figure 5. The device can be anticipated to be removably adapted to a cast and fitted with a battery charging unit so that the patient can effect recharging, should this be desirable, with reserve batter backs or during periods of non-treatment. Further, circuitry capable of tracking the length of time, or dose, of treatment, as well as visual or audible alarms to inform the patient of the same, are readily incorporated into the device.

6

EP 0 252 594 B1

TABLE I

$$f_B = \frac{1}{T}$$

| PHYSICAL EFFECT | DEFINING EQUATION | $\tau$ TIME CONSTANT | EQUIVALENT BREAK FREQUENCY | EQUIV. $\Delta t_{PW}$ | PARAMETERS |
|---|---|---|---|---|---|
| MAGNETIC DIFFUSION | $\frac{\delta \overline{H}}{\delta t} = \frac{1}{\mu \sigma} \nabla^2 H$ | $S^2 \mu \sigma$ | $\frac{2}{(25 \times 10^{-4}) \times 4\pi \times 10^{-7}} \approx 100$ MHz | | $\mu$ = permeability $\sigma$ = conductivity $H$ = field |
| ELECTRIC DIFFUSION | $\nabla \cdot \sigma \overline{E} = \frac{\delta \rho}{\delta t}$ | $\frac{\varepsilon}{\sigma}$ | $\frac{1}{10^{-9},10^6} \approx 1$ MHz – 1 GHz | $0.5\mu s$ | $\varepsilon$ = permitivity $\mu$ = permeability |
| VISCOUS DIFFUSION | $\frac{d\overline{V}}{dt} = \frac{n}{\rho} \nabla^2 \overline{V}$ | $\frac{\rho R^2}{n}$ | $\frac{10^{-3}}{10^3 (10^{-6})^2} \approx 1$ MHz | $5\mu S$ | $n$ = viscosity $\rho$ = density $R$ = channel radius $(1\mu m)$ |
| MECHANICAL | | $\frac{\delta^2}{mk}$ | $\frac{.5 \times 10^{\pm 6} \times 10^{-15}}{10^3 (10^{-6})^2} \approx 500$ Hz | | $m$ = modulus $k$ = HYD Constant |

EP 0 252 594 B1

TABLE  II  - STIFFNESS RATIOS

PULSE WIDTH (µs) @ 100 mV

| EXPERIMENT | CONTROL | 2 | 3 | 4 | 5 | 7 | 10 |
|---|---|---|---|---|---|---|---|
| 9<br>May | $\langle X\rangle$ = 1.20<br>SD = 0.78<br>N = 6 | | | | | | $\langle X\rangle$ = .366<br>SD = .194<br>N = 9 |
| 11<br>June<br>July | $\langle X\rangle$ = .236<br>SD = .147<br>N = 7 | | | | $\langle X\rangle$ = .312<br>SD = .175<br>N = 18 | | |
| 16<br>Oct. -<br>Nov. | $\langle X\rangle$ = .244<br>SD = .114<br>N = 4 | | | | $\langle X\rangle$ = .302<br>SD = .166<br>N = 5 | | |
| 18<br>Nov. -<br>Dec. | $\langle X\rangle$ = .207<br>SD = .009<br>N = 2 | $\langle X\rangle$ = .225<br>SD = .097<br>N = 6 | | | $\langle X\rangle$ = .216<br>SD = .156<br>N = 6 | | |
| 19<br>Jan. | $\langle X\rangle$ = .156<br>SD = .029<br>N = 5 | $\langle X\rangle$ = .265<br>SD = .204<br>N = 9 | | $\langle X\rangle$ = .156<br>SD = .029<br>N = 5 | | | |
| 22<br>Feb. | $\langle X\rangle$ = .156<br>SD = .030<br>N = 6 | | | | | | $\langle X\rangle$ = .301<br>SD = .043<br>N = 7 |

TABLE III - STIFFNESS RATIO

| EXPERIMENT | CONTROL | 10 | 25 | 50 | 75 | 100 | 125 | 200 |
|---|---|---|---|---|---|---|---|---|
| 9<br>May | $\bar{x}$ = .120<br>σ = .078<br>N = 6 | | | | | | | $\bar{x}$ = .266<br>σ = .194<br>N = 16 |
| 10<br>June | $\bar{x}$ = .128<br>σ = .044<br>N = 6 | | | | | | | $\bar{x}$ = .142<br>σ = .086<br>N = 9 |
| 11<br>June-<br>July | $\bar{x}$ = .236<br>σ = .147<br>N = 7 | | | | | $\bar{x}$ = .312<br>σ = .175<br>N = 18 | | |
| 12.5<br>July-<br>August | $\bar{x}$ = .201<br>σ = .097<br>N = 3 | | | $\bar{x}$ = .464<br>σ = .074<br>N = 7 | | | | |
| 13.5<br>August | $\bar{x}$ = .224<br>σ =<br>N = 1 | | | $\bar{x}$ = .214<br>σ = .079<br>N = 5 | | | | |
| 14<br>September | $\bar{x}$ = .226<br>σ = .107<br>N = 5 | $\bar{x}$ = .189<br>σ = .116<br>N = 11 | $\bar{x}$ = .113<br>σ = .072<br>N = 3 | | | | | |
| 15<br>September-<br>October | $\bar{x}$ = .167<br>σ = .076<br>N = 4 | | $\bar{x}$ = .177<br>σ = .066<br>N = 8 | $\bar{x}$ = .275<br>σ = .098<br>N = 6 | $\bar{x}$ = .327<br>σ = .148<br>N = 6 | | | |
| 15.5<br>October | $\bar{x}$ = .288<br>σ = .104<br>N = 2 | | | | $\bar{x}$ = .272<br>σ = .201<br>N = 8 | | | |
| 16<br>October-<br>November | $\bar{x}$ = .244<br>σ = .114<br>N = 4 | | | | $\bar{x}$ = .295<br>σ = .139<br>N = 7 | $\bar{x}$ = .302<br>σ = .166<br>N = 5 | $\bar{x}$ = .359<br>σ = .237<br>N = 8 | |

TABLE IV

| Battery Type | $V_{E min}$ | N | US $\Delta t_{pw}$ | mA $I_{BA}$ | Cap mA-hrs | Dur Hrs | Wt g |
|---|---|---|---|---|---|---|---|
| 12-2/3 AA NiCAD | 13.2 | 20 | 5 US | 16.4 | 300 | 18.3 | 148 |
| 15 2/3 AA NiCAD | 16.5 | 26 | 5 US | 8.7 | 300 | 34.4 | 1858 |
| 3x9V NiCAD | 23.1 | 37 | 5 US | 3.7 | 100 | 27 | 138 |
| 2x9V Alkaline | 12 | 19 | 5 US | 21.6 | 450 | 21 | 92 |
| 3x9V | 18 | 28 | 5 US | 6.9 | 450 | 65 | 138 |
| P.P. Lithium (2) | 10 | 15 | 5 US | 37.2 | 1300 | 35 | 68 |
| 6 2/3A Lithium | 15 | 23 | 5 US | 11.4 | 916 | 80 | 81 |
| "9V" Zn AIR | 13 | 20 | 5 US | 17.1 | 700 | 41 | 60 |
| 9 2/3 Lithium | 22.5 | 35 | 7 | 7.1 | 916 | 129 | |
| 15 2/3AA NiCAD | 16.5 | 26 | 7 US | 16.6 | 300 | 18.1 | |
| 3 - "9V" NiCAD | 23.1 | 37 | 7 US | 6.65 | 100 | 15 | |
| 3 - "9V" Alkaline | 18 | 28 | 7 US | 13 | 450 | 35 | |
| 2 - "9V" Zn-AIR | 13 | 20 | 7 US | 33 | 700 | 21 | |
| 6 - 9V Alkaline | 36 | 57 | 7 | 2.4 | 450 | 188 | |
| 2 - 9V Alkaline | 12 | 19 | 3 US | 8.1 | 450 | 56 | |
| 3 - 9V Alkaline | 18 | 28 | 3 US | 2.9 | 450 | 157 | |
| 6 - 2/3A Lithium | 15 | 23 | 3 US | 4.5 | 916 | 204 | |
| 15 - 2/3 AA NiCAD | 16.5 | 26 | 3 US | 3.5 | 300 | 86 | |
| 6 - 2/3A Lithium | 15 | 23 | 10 | 44 | 916 | 21 | |
| 9 - 2/3A Lithium | 27.5 | 36 | 10 | 13.9 | 916 | 66 | |
| 3 - PP Lithium | 15 | 23 | | 44 | 1300 | 30 | |

## Claims

1. A portable apparatus for electro-therapy of bone fractures and for stimulating tissue healing at a treatment site, said apparatus having a source of electric power connected to means for converting the electric power into a series of amplitude-symmetric voltage cycles and transducer means for receiving said symmetric voltage cycles characterized in that:

   the voltage cycles comprise a train of bursted pulses having a pulse width of 0.5 to 20 microseconds; the burst frequency is in the range of 5-25Hz;

   the burst width is 1-10 milliseconds; and,

   the transducer means is positionable so that an electromagnetic field at the treatment site is generated by applying the electric power to the transducer.

**2.** Apparatus according to claim 1, wherein the source of electric power is a battery having a voltage of 10-40 volts and a volume of about 32.77 to 98.32 cubic centimetres (2-6 cubic inches).

**3.** Apparatus according to claim 1 or 2, wherein said source of electric power weighs less than 0.91 kilograms (2 pounds).

**4.** Apparatus according to any one of the preceding claims, wherein the transducer means is a coil transducer selected from the group consisting of a conformal solenoid, oblique coil, and simple coil.

**5.** Apparatus according to claim 1, wherein the transducer means is a conformal solenoid.

**6.** Apparatus according to claim 1, wherein the transducer means is a conformal magnetic dipole.

**7.** Apparatus according to claim 1, further comprising a voltage cycle having a peak to peak amplitude of greater than 25mV and less than 200mV.

**8.** Apparatus according to claim 1, wherein the transducer means is formed in a cast whereby the transducer means is positionable closely proximal to a fracture or treatment site.

**9.** Apparatus according to claim 1, wherein the electric pulses are sinusoidal.

**10.** Apparatus according to claim 1, wherein the electric pulses are rectangular.

**11.** Apparatus according to claim 1, wherein the pulse wave form is triangular.

**12.** Apparatus according to claim 1, wherein the train of bursted pulses has a peak to peak amplitude of 50-150mV.

**Patentansprüche**

**1.** Tragbarer Apparat zur Elektro-Therapie von Knochenbrüchen und zur Anregung der Gewebeheilung an einer Behandlungsstelle des Körpers, mit einer Stromquelle, die mit Mitteln versehen ist zur Umwandlung elektrischer Energie in jeweils eine Serie amplituden-symmetrischer Spannungs-Zyklen, und mit Übertragungsmitteln zur Aufnahme bzw. Übertragung der symmetrischen Spannungs-Zyklen, gekennzeichnet durch folgende Merkmale:
Die Spannungs-Zyklen umfassen bzw. bestehen aus einer Folge von Einzel-Pulsen mit einer Pulsweite von je $At_{pW}$ = 0,5 bis 20 Mikrosekunden;
die Frequenz, mit der Pulsfolgen aufeinander folgen, liegt im Bereich von 5 - 25 Hz;
die Dauer der Pulsfolgen $\Delta t_{BW}$ beträgt 1 - 10 Millisekunden; und die Übertragungsmittel sind derart (am Körper) positionierbar, daß durch Anlegen der elektrischen Energie an den Übertrager ein elektromagnetisches Feld an der Behandlungsstelle erzeugt wird.

**2.** Apparat gemäß Anspruch 1, wobei die elektrische Stromquelle eine Batterie mit einer Spannung von 10 - 40 Volt und ein Volumen von ungefähr 32,77 - 98,32 cm$^3$ (2 bis 6 Kubik-Inches) hat.

**3.** Apparat nach Ansprüchen 1 oder 2, wobei die Stromquelle weniger als 0,91 kg (2 pounds) wiegt.

**4.** Apparat nach einem den vorhergehenden Ansprüche, wobei die Übertragungsmittel eine Übertragungsspule ist, ausgewählt aus der Gruppe, bestehend aus winkeltreuen Spulen, schrägen Spulen und einfachen Spulen.

**5.** Apparat nach Anspruch 1, wobei der Übertrager eine winkeltreue (konformale) Spule ist.

**6.** Apparat nach Anspruch 1, wobei der Übertrager ein winkeltreuer magnetischer Dipol ist.

**7.** Apparat nach Anspruch 1, wobei die Spitzen-Amplitude des Spannungs-Zyklus größer als 25 mV und kleiner als 200 mV ist.

**8.** Apparat nach Anspruch 1, wobei der Übertrager durch Guß gebildet und der Übertrager nahe an der Bruchstelle positionierbar ist.

**9.** Apparat nach Anspruch 1, wobei die elektrischen Pulse sinusförmig sind.

**10.** Apparat nach Anspruch 1, wobei die elektrischen Pulse rechtwinklig sind.

**11.** Apparat nach Anspruch 1, wobei die Pulse Dreieckform aufweisen.

**12.** Apparat nach Anspruch 1, wobei die Pulsfolge eine Amplitude von 50 - 150 mV hat.

**Revendications**

**1.** Appareil portable pour l'électrothérapie de fractures des os et pour stimuler la cicatrisation des tissus à un site de traitement, ledit appareil comprenant une source d'énergie électrique connectée à des moyens de conversion de l'énergie électrique en une série de cycles de tension d'amplitude symétrique, et des moyens de transduction qui reçoivent lesdits cycles de tension symétrique, caractérisé en ce que :
les cycles de tension comprennent un train d'impulsions en salve, ayant une largeur d'impulsion de 0,5 à 20 $\mu$s ;
la fréquence des salves est de l'ordre de 5 à 25 Hz ;
la largeur des salves est de 1 à 10 ms ; et
on peut positionner les moyens de transduction de sorte qu'un champ électromagnétique est engendré au site de traitement par application de l'énergie électrique aux moyens de transduction.

**2.** Appareil suivant la revendication 1, dans lequel la source d'énergie électrique est une batterie ayant une tension de 10 à 40 volts et un volume de 32,77 à 98,32 cm$^3$ environ.

**3.** Appareil suivant la revendication 1 ou 2, dans lequel ladite source d'énergie électrique a une masse inférieure à 0,91 kg.

**4.** Appareil suivant l'une quelconque des revendications précédentes, dans lequel les moyens de transduction sont un transducteur à enroulement choisi dans le groupe comprenant un solénoïde conformable, un enroulement oblique et un enroulement simple.

**5.** Appareil suivant la revendication 5, dans lequel les moyens detransduction sont un solénoïde conformable.

**6.** Appareil suivant la revendication 1, dans lequel les moyens de transduction sont constitués par un dipôle magnétique conformable.

**7.** Appareil suivant la revendication 1, comprenant en outre un cycle de tension qui a une amplitude de crête à crête supérieure à 25 mV et inférieure à 200 mV.

**8.** Appareil suivant la revendication 1, dans lequel les moyens de transduction sont formés dans un moulage de sorte que les moyens de transduction peuvent être placés très près d'un site de fracture.

**9.** Appareil suivant la revendication 1, dans lequel les impulsions électriques sont sinusoïdales.

**10.** Appareil suivant la revendication 1, dans lequel les impulsions électriques sont rectangulaires.

**11.** Appareil suivant la revendication 1, dans lequel la forme d'onde des impulsions est triangulaire.

**12.** Appareil suivant la revendication 1, dans lequel le train d'impulsions en salue a une amplitude de crête à crête de 50 à 150 mV.

$V_{s-c}$  $\Delta t_{PW}$  $\Delta t_{BW}$

$T_{BW}$  $f_{BW} = \dfrac{1}{T_{BW}}$

FIG. 1

| COIL TYPE: | SIMPLE | OBLIQUE | SOLENOID | HELMHOLTZ |
|---|---|---|---|---|
| | | | | |
| LIMB DIAMETER | 5 | 5 | 5 | 5 |
| $L(H)$ | $\dfrac{\pi SN^2 \times 10^{-7}}{2 \times 9.8}$ | $\dfrac{\pi \sqrt{3/2}\, SN^2 \times 10^{-7}}{2 \times 9.8}$ | $\dfrac{6\pi SN^2 \times 10^{-7}}{2\sqrt{2} \times 9.8}$ | $\dfrac{\pi SN^2 \times 1.1 \times 10^{-7}}{2\sqrt{2} \times 9.8}$ |
| $f_c \left(\dfrac{GAUSS}{AMP}\right)$ | $\dfrac{2\pi N}{5S}$ | $\dfrac{2\pi N}{5S\sqrt{3/2}}$ | $\dfrac{\pi N\sqrt{2}}{5S}$ | $\dfrac{\pi N\sqrt{2}}{5S\sqrt{3/2}}\,^{3/2}$ |
| $K_c$ | $1.02 \times 10^{-8} S^3$ | $1.87 \times 10^{-8} S^3$ | $0.861 \times 10^{-8} S^3$ | $5.33 \times 10^{-8} S^3$ |
| NORMALIZED | 1.18 | 2.17 | 1.0 | 6.19 |

FIG. 3

EP 0 252 594 B1

FIG. 2

EP 0 252 594 B1

FIG. 6

FIG. 7

FIG. 8

FIBULA

MUSCLE

SOLENOID
COIL WINDINGS
(N-TURNS)

PLASTER
CAST

POWER
SUPPLY

DRIVING
SIGNAL
CIRCUITS

TIBIA (FRACTURE)

FIG. 4

BATTERY
POWER
SUPPLY

SIGNAL GENERATOR
& DRIVER AMPLIFIER,
TIMER, CALIBRATION
& STATUS CIRCUITS

TRANSDUCER

BATTERY
CHARGER

FIG. 5

FIG. 9